# EUROPEAN PATENT APPLICATION

(11) **EP 2 031 053 A1**
(43) Date of publication of application: **04.03.2009**
(21) Application number: 07115404.1
(22) Date of filing: 31.08.2007
(51) Int. Cl.: C12N 1/21, C12P 19/26

(54) **Production of HA**

(71) Applicant: THE UNIVERSITY OF QUEENSLAND, St Lucia, QLD 4072 (AU); Sugar Industry Innovation Pty Ltd, St. Lucia, QLD 4072 (AU)
(72) Inventor: The designation of the inventor has not yet been filed
(74) Representative: Jones, Stephen Anthony

(57) **Abstract**

A method for producing hyaluronic acid is described which method comprises growing *Streptococcus* cells in a culture medium, which cells express the enzymes required for hyaluronic acid synthesis, wherein the activity in the cells of one or more enzymes selected from UDP-glucose dehydrogenase, phosphoglucoisomerase, glucosamine-6-P acetyl transferase/N-acetyl glucosamine-1-phosphate pyrophosphorylase and phosphoglucomutase has been increased; and optionally recovering the hyaluronic acid produced by the cells.

## Description

### Field of the invention

The present invention relates to methods for the production of hyaluronic acid in *Streptococcus* sp., as well as to high molecular weight hyaluronic acid produced by such a method.

### Background to the invention

Hyaluronic acid (HA) is a uniformly repetitive, linear glycosaminoglycan composed of 2,000-25,000 disaccharides of glucuronic acid and N-acetylglucosamine joined alternately by β-1-3 and β-1-4 glycosidic bonds: [β-1,4- glucuronic acid-β-1,3-N-acetyl glucosamine-]ₙ.

Reflecting its variety of natural functions, HA has found a number of applications in medicine, cosmetics and speciality foods. In many applications, high molecular weight is desired property and different approaches have been employed to produce high molecular weight (MW) HA.

High MW HA can be obtained through careful extraction from rooster comb. HA in rooster combs may reach very high values, for instance up to 12-14 million Dalton (Da). Depending on the extraction process, a final product of 3-5 MDa can be obtained. Increased reticence to the use of animal derived products in medicine and cosmetics have seen a shift towards microbial HA production. Microbial HA production through fermentation of group C streptococci, in particular *Streptococcus equi* subsp. *equi* and S. *equi* subsp. *zooepidemicus,* has been practised commercially since the early 1980s. Microbial HA, however, is of lower molecular weight (typically 0.5 to 2 MDa) than HA obtainable from rooster comb.

In some applications, chemical cross-linking has been used to increase molecular weight (e.g., U.S. Pat. No. 4,582,865; U.S. Pat. No. 6,903,199; U.S. Pat. No. 7,125,860; and U.S. Pat. No. 6,703,444). In other applications, notably ophthalmic applications, cross-linking is undesirable and strain engineering is the only means of realising high MW HA.

HA is synthesised as an extracellular capsule by pathogenic Lancefield group A and C streptococci. Under the microscope, these non-sporulating and nonmotile bacteria appear as spherical or ovoid cells that are typically arranged in pairs or chains surrounded by an extensive extracellular capsule. On sheep blood agar plates, colonies of these β-hemolytic bacteria will produce a clear zone with HA identified as a mucoid or slimy translucent layer surrounding bacterial colonies. The HA capsule is a virulence factor in these streptococci, presumably affording the bacterium a stealth function as the immune system of higher organisms fails to recognise the HA capsule as a foreign entity

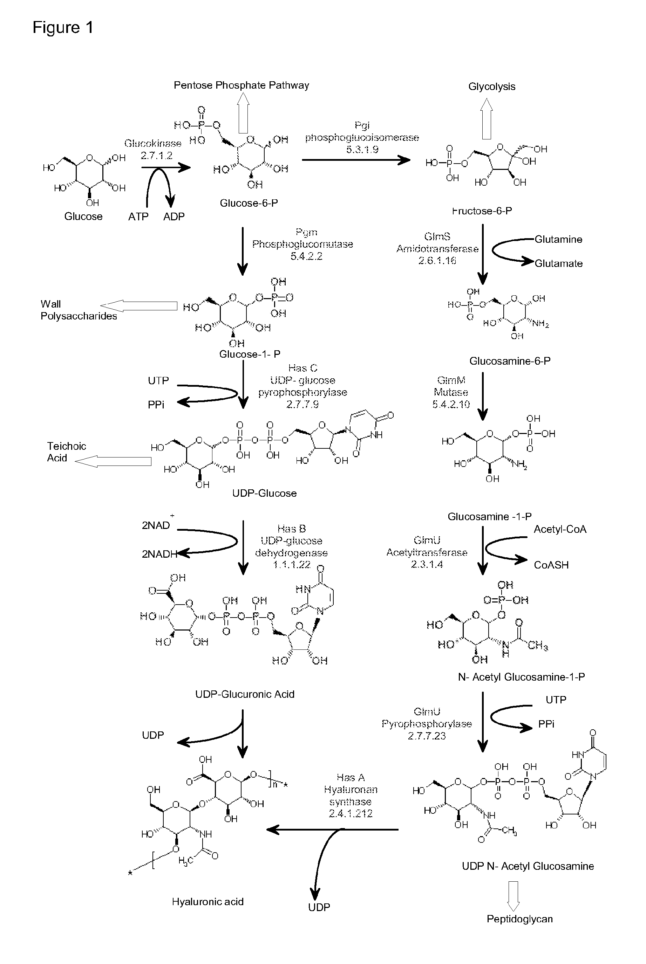

The HA biosynthetic pathway involves two groups of reactions (Figure 1). The first group of reactions starts from glucose-6-phospahate catalysed by phosphoglucoisomerase (EC 5.3.1.9) into fructose-6-phospahte leading into 2 branches. The first branch of the pathway, leaving the glucose-6-phosphate node is a common step in the production of storage polysaccharides in many organisms. α-Phosphoglucomutase (EC 5.4.2.2) converts glucose-6-phosphate to glucose-1-phosphate in a reversible reaction. UDP-glucose pyrophosphorylase (EC 2.7.7.9) catalyses the reaction of UTP and glucose-1-phosphate to produce the nucleotide sugar UDP-glucose. UDP-glucuronic acid is then obtained by specific oxidation of the primary alcohol group of UDP-glucose through the action of UDP-glucose dehydrogenase (EC 1.1.1.22). The pathway originating from fructose-6-phosphate is involved in the production of amino sugars. Amino group transfer from glutamine to fructose-6-phosphate by an amidotransferase (EC 2.6.1.16) yields glucosamine-6-phosphate. Phosphate group rearrangement by a mutase (EC 5.4.2.10) generates glucosamine-1-phosphate from glucosamine-6-phosphate. Acetyl group transfer by an acetyltransferase (EC 2.3.1.4) forms N-acetyl glucosamine-6-phosphate. This is an energy-consuming step since hydrolysis of the thioester bond in acetyl-CoA liberates the energy equivalent of ATP hydrolysis. Finally, a pyrophosphorylase (EC 2.7.7.23) adds UDP to obtain UDP-N-acetylglucosamine. The participation of UTP in these reactions generates activated glycosyl donors that can be polymerised into HA by HA synthase (EC 2.4.1.212).

The HA synthase plays an important role in controlling HA MW and site directed mutagenesis has been employed to increase HA MW (Kumari, K., et al. (2006). "Mutation of Two Intramembrane Polar Residues Conserved within the Hyaluronan Synthase Family Alters Hyaluronan Product Size." J. Biol. Chem. 281(17): 11755-11760). Random mutagenesis followed by strain selection has also been used to improve strain properties including HA MW (Kim, J.-H., et al. (1996). "Selection of a Streptococcus equi mutant and optimization of culture conditions for the production of high molecular weight hyaluronic acid." Enzy. Microbial Tech. 19(6): 440-445; Lee, M.S., et al. (1999). "Construction and analysis of a library for random insertional mutagenesis in Streptococcus pneumoniae: Use for recovery of mutants defective in genetic transformation and for identification of essential genes." Appl. Environ. Microbiol. 65(5): 1883-1890; U.S. Pat. No. 5,496,726).

### Summary of the invention

We have now surprisingly found that enhanced expression in streptococci of particular enzymes involved in the biosynthesis of the HA precursors leads to an increase in the molecular weight of the HA produced. Previous attempts to improve the biosynthesis of HA have focussed on HA synthase, which catalyses the final step in the synthesis of HA. However we have found that cells that have been engineered to express enhanced levels of UDP-glucose dehydrogenase, phosphoglucoisomerase, glucosamine-6-phosphate acetyl transferase/N-acetyl glucosamine-1-phosphate pyrophosphorylase (a bifunctional enzyme) or phospho-glucomutase produce HA with a significant increase in molecular weight compared with wildtype cells and cells with increased expression of HA synthase or UDP-glucose pyrophosphorylase.

Accordingly, the present invention provides a method for producing hyaluronic acid which method comprises growing *Streptococcus* cells in a culture medium, which cells express the enzymes required for hyaluronic acid synthesis, wherein the activity in the cells of one or more enzymes selected from UDP-glucose dehydrogenase, phosphoglucoisomerase, glucosamine-6-phosphate acetyl transferase/N-acetylglucosamine-1-phosphate pyrophosphorylase and phosphoglucomutase has been increased; and optionally recovering the hyaluronic acid produced by the cells.

In a related aspect, the present invention provides a method for producing hyaluronic acid which comprises recovering hyaluronic acid from *Streptococcus* cells that express the enzymes required for hyaluronic acid synthesis, wherein the activity in the cells of one or more enzymes selected from UDP-glucose dehydrogenase, phosphoglucoisomerase, glucosamine-6-phosphate acetyl transferase/N-acetylglucosamine-1-phosphate pyrophosphorylase and phosphoglucomutase has been increased.

The present invention also provides a method for producing hyaluronic acid which method comprises growing *Streptococcus* cells in a culture medium, which cells express the enzymes required for hyaluronic acid synthesis, wherein the cells have been engineered or treated to increase the activity in the cells of one or more enzymes selected from UDP-glucose dehydrogenase phosphoglucoisomerase, glucosamine-6-phosphate acetyl transferase/N-acetyl glucosamine-1-phosphate pyrophosphorylase and phosphoglucomutase; and optionally recovering the hyaluronic acid produced by the cells.

In a related aspect, the present invention provides a method for producing hyaluronic acid which comprises recovering hyaluronic acid from *Streptococcus* cells that express the enzymes required for hyaluronic acid synthesis, wherein the cells have been engineered or treated to increase the activity in the cells of one or more enzymes selected from UDP-glucose dehydrogenase phosphoglucoisomerase, glucosamine-6-phosphate acetyl transferase/N-acetyl glucosamine-1-phosphate pyrophosphorylase and phosphoglucomutase.

The present invention further provides hyaluronic acid obtained or obtainable by the method of the invention, having an average molecular weight of at least 3 or 3.5 MDa, the hyaluronic acid being substantially non-crosslinked.

In another aspect, the present invention provides a streptococcal cell which comprises the enzymes for synthesis of hyaluronic acid, which cell has been genetically modified to overexpress one or more enzymes selected from UDP-glucose dehydrogenase, phosoglucoisomerase, glucosamine-6-phosphate acetyl transferase/N-acetylglucosamine-1-phosphate pyrophosphorylase and phosphoglucomutase.

### Detailed description of the invention

Unless defined otherwise, all technical and scientific terms used herein have the same meaning as commonly understood by one of ordinary skill in the art (e.g. in cell biology, chemistry, molecular biology and cell culture). Standard techniques used for molecular and biochemical methods can be found in Sambrook et al., Molecular Cloning: A Laboratory Manual, 3rd ed. (2001) Cold Spring Harbor Laboratory Press, Cold Spring Harbor, N.Y. and Ausubel et al., Short Protocols in Molecular Biology (1999) 4th Ed, John Wiley & Sons, Inc. - and the full version entitled Current Protocols in Molecular Biology).

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Throughout this specification, reference to numerical values, unless stated otherwise, is to be taken as meaning "about" that numerical value. The term "about" is used to indicate that a value includes the inherent variation of error for the device and the method being employed to determine the value, or the variation that exists among the study subjects.

The present invention is based on the finding that increased expression/activity of a number of enzymes in the pathway for hyaluronic acid production in *Streptococcus* sp. leads to an increase in the molecular weight (MW) of the resulting hyaluronic acid produced by the cells. The specific enzymes identified as giving rise to an increase in HA MW are: UDP-glucose dehydrogenase (HasB
- EC 1.1.1.22), phosphoglucoisomerase (HasE, Pgi - EC 5.3.1.9), glucosamine-6-phosphate acetyl transferase/ N-acetylglucosamine-1-phosphate pyrophosphorylase (HasD, GlmU - EC 2.3.1.4 and 2.7.7.23) and phosphoglucomutase (Pgm - EC 5.4.2.2).

Thus in the methods of the invention, the streptococcus cells have increased activity/expression of one or more enzymes selected from UDP-glucose dehydrogenase, phosphoglucoisomerase, glucosamine-6-phosphate acetyl transferase/N-acetylglucosamine-1-phosphate pyrophosphorylase and phosphoglucomutase. Preferably the cells have increased activity/expression of at least phosphoglucoisomerase or phosphoglucomutase, more preferably at least phosphoglucoisomerase.

In one embodiment, cells have wild type levels and activity of HA synthase (HasA).

Increased expression/activity is measured relative to an equivalent wild-type strain which has not been genetically modified and which is grown under standard conditions (37°C in rich media (M17G) or chemically defined media (CDM) supplemented with 2% w/v D-glucose). E.g. in the case of mucoid Group *C Streptococcus equi* subsp. *zooepidemicus,* a suitable control strain is ATCC 35246.

In one embodiment, increased activity of the enzymes is effected by genetically engineering the cells by introducing one or more nucleic acid sequences that direct expression of the enzymes. Such sequences can be introduced by various techniques known to persons skilled in the art, such as the introduction of plasmid DNA into cells using electroporation followed by subsequent selection of transformed cells on selective media. These heterologous nucleic acid sequences may be maintained extrachromosomally or may be introduced into the host cell genome by homologous recombination.

Accordingly, the present invention provides a streptococcal cell which comprises the enzymes for synthesis of hyaluronic acid, which cell has been genetically modified to overexpress one or more enzymes selected from UDP-glucose dehydrogenase, phosoglucoisomerase, glucosamine-6- phosphate acetyl transferase/N-acetylglucosamine-1-phosphate pyrophosphorylase and phosphoglucomutase. In a particular embodiment, the cell comprises one or more heterologous nucleic acid sequences encoding one or more of the enzymes. In another embodiment, the cells comprise one or more mutations in genomic regulatory sequences encoding the one or more enzymes, which mutations result in increased levels of expression of the one or more enzymes, relative to a wild type cell. In a further embodiment, the cells may comprise one or more mutations in the coding sequences of the one or more enzymes that give rise to increased enzyme activity. Combinations of these embodiments are also possible.

It is particularly preferred in relation to the methods and cells described above, that the cells have increased activity/expression of phosoglucoisomerase, glucosamine-6-phosphate acetyl transferase/N-acetylglucosamine-1-phosphate pyrophosphorylase and/or phosphoglucomutase, more preferably phosoglucoisomerase and/or phosphoglucomutase.

Nucleic acid sequences encoding the enzymes of interest, operably linked to regulatory sequences that are capable of directing expression of the enzymes in a suitable streptococcal host cell, can be derived from a number of sources. The HAS operons from four streptococcal species have been cloned to date. The sequence of hasB has been cloned for S. *pyogenes, S. uberis, S. equisimilus* and *S*. *equis* subsp. *zooepidemicus.* The sequence of hasD/glmU has been cloned for S. *equisimilus* and S. *equis* subsp. *zooepidemicus.* Further, the sequence of hasE/pgi has been cloned for S. *equis* subsp. *zooepidemicus.* Sequences can also be obtained from other species, e.g. *B. subtilis* has a homologue of hasB termed tuaD. HasD/glmU has been cloned for a variety of bacterial species e.g. *S. pyogenes* (Accession No. YP_001129027); *E*. *coli* (Accession Nos. ABG71900 and POACC7) and *B. subtilis* (Accession No. P14192). Pgm has also been cloned for a variety of bacterial species e.g. *S. pyogenes* (Accession No. YP_596772); *E*. *coli* K12 (Accession No. AAC73782) and B. *subtilis* (ybbT) (Accession No. CAB11953). The complete genomes of S. *pyogenes, E. coli* and *B. subtilis* have been sequenced and published.

By way of a further example, suitable oligonucleotide primers for amplifying hasB, hasD (glmU), hasE (pgi) and pgm sequences from *S. zooepidemicus* genomic DNA are described in the experimental section below.

In some embodiments, the nucleic acid sequences encoding one or more of the enzymes of interest are operably linked to regulatory sequences that are inducible so that expression of the enzymes upregulated as desired, by the addition of an inducer molecule to the culture medium.

An alternative approach is to modify the host cell's regulatory sequences that control expression of the endogenous sequences encoding the enzymes of interest by homologous recombination, e.g. promoter sequences.

A further approach is to treat the cells such that amplification of the endogenous sequences occurs, resulting in increased copy number of the endogenous DNA encoding the enzymes of interest, leading to increased expression and activity of the enzymes.

It is also possible to subject cells to various mutagenesis treatments and to test for increases in enzyme activity using enzyme assays known in the arts, examples of which are described in the experimental section. It is also possible to use site-directed mutagenesis to modify the coding sequence of the enzymes to increase enzyme activity.

The activity of the enzymes of interest can also be upregulated using chemical treatments, e.g. molecules that upregulate expression of one or more of the enzymes of interest e.g. compounds that bind to transcriptional regulatory proteins and modify the binding of the transcriptional regulatory proteins to the regulatory sequences controlling expression of the enzymes of interest. Suitable compounds can be identified, for example, by screening compound libraries and testing for increases in enzyme activity as discussed above.

The streptococcus cells of the invention, and for use in the methods of the invention are preferably Lancefield group A or group C streptococci, such as *Streptococcus equi* (for example *Streptococcus equi* subsp. *zooepidemicus or Streptococcus equi* subsp. equi). These bacteria naturally produce HA as an extracellular capsule.

HA is produced according to the method of the invention by culturing suitable streptococci, such as are described above, under suitable conditions. Either continuous fermentation or a batch fed process can be used. Examples of conditions that can be used to produce HA are described in WO92/08777, which describes a continuous fermentation process with a pH of from 6.0 to 7.0 and dissolved oxygen at less than 1% saturation. US 6,537,795 describes a batch fed process. A chemically defined media suitable for the culture of cells is described in the experimental section. Cells are typically cultured at from 35°C to 40°C, such as at about 37°C.

Once the desired level of HA production has occurred in batch, or at a suitable interval for continuous culture, HA can then be recovered from the cells. A number of methods for purifying HA from bacteria are known in the art. The HA is typically subject to one or more purification steps, particularly where medical grade HA is been produced. The following description, based on US Patent No. 4,782,046, is by way of example:

Typically the biomass is killed with a suitable agent such as formaldehyde and the HA extracted with an anionic surfactant, such as sodium lauryl sulfate (SLS) or sodium dodecyl sulphate (SDS), or an equivalent anionic detergent, to release the HA from the cells.

The resulting mixture may then simply be filtered, for example through a 0.45 µm mixed cellulose esters filter. An alternative is to treat the mixture with a non-ionic detergent, such as hexadecyltrimethylammonium bromide, or equivalent non-ionic detergent, to precipitate HA and the anionic detergent. The resulting precipitate can be collected via centrifugation or sieve filtration. This precipitate is then solubilised in CaCl₂. The resulting suspension is centrifuged or sieve filtered to remove the precipitate which contains cellular contaminants and both detergents.

The filtrate/supernatant from either method is then extracted with a suitable alcohol (95% EtOH or 99% isopropanol preferred). A gelatinous precipitate forms which is collected via centrifugation or sieve filtration. The pellet is typically washed, for example with an ethanol/saline solution.

Additional purification steps, as described in US Patent No. 4,782,046, that may be used are as follows: the precipitate is solubilised overnight at 4°C to 10°C in deionised, distilled water. The suspension is centrifuged or sieve filtered to remove the precipitate. 1% w/v NaCl is added to the supernatant and dissolved. Then, an appropriate alcohol is added to reprecipitate the HA. Such precipitate is allowed to settle after which it is collected via centrifugation or sieve filtration.

The solubilisation of the HA in water followed by 1.0% NaCl addition and alcohol precipitation may be repeated in increasingly smaller volumes (1/20-1/100 original volume) until the HA-water solution is clear. This may require at least four additional alcohol precipitation steps.

The resulting HA may be sterilised using, for example, 0.1% betapropiolactone (4°C to 10°C at 24-48 hours) - the betapropiolactone subsequently being hydrolysed by heating at 37°C.

Other sterilisation methods include filtration using, for example, a suitable protein-binding filter, such as a mixed cellulose esters filter, typically with a pore size of about 0.45 um.

The resulting bacterial HA of the invention preferably has a MW of more than 3 MDa, preferably more than 3.5 MDa (without being subject to crosslinking).

The HA of the invention can be used in a variety of applications, such as in cosmetic and reconstructive surgery; in skin anti-ageing, anti-wrinkle products; for replacing biological fluids including synovial fluid (e.g. as an injectable formulation for treating osteoarthritis); for the topical treatment of burns and ulcers; as a surgical aid in cataract extraction, IOL implantation, corneal transplantation, glaucoma filtration, and retinal attachment surgery (e.g. in the form of eye drops or a gel); for adhesion management in surgery, e.g. cardiac surgery, hernia repair, nasal/sinus repair, arthroscopic surgery and spinal surgery; and the like.

Accordingly the present invention further comprises cosmetic compositions comprising purified bacterial HA obtained or obtainable by the methods of the invention, together with a cosmetically acceptable carrier or diluent, as well as pharmaceutical compositions comprising purified bacterial HA obtained or obtainable by the methods of the invention, together with a pharmaceutically acceptable carrier or diluents.

The present invention will now be further described with reference to the following examples, which are illustrative only and non-limiting.

### EXAMPLES

### Materials and methods

### Bacterial strain

The mucoid Group C *Streptococcus equi* subsp. *zooepidemicus* strain ATCC 35246 (*S. zooepidemicus*) was obtained from the American Type Culture Collection (Rockville, MD, USA).

### Construction of recombinant strains

The 6 genes, namely *hasA, hasb, hasC, glmU, pgm* and *pgi* were amplified from *S*. *zooepidemicus* genomic DNA using the primers listed in Table 1. Oligonucleotide primers were designed based on data available from the partial sequence of the *Streptococcus equi* subspecies *zooepidemicus* (*S. zooepidemicus*) has operon available on NCBI (ncbi.nlm.nih.gov: Accession number AF347022). Primer GuaB forward and reverse amplify a housekeeping gene of *S*. *zooepidemicus* and was used as a polymerase chain reaction (PCR) positive control for *S*. *zooepidemicus.* The PCR product sizes were confirmed an agarose gel and the bands extracted using QIAquick Gel Extraction kit (Qiagen). The purified PCR products were double digested with the desired restriction enzymes (see Table 1) and ligated into the nisin inducible plasmid pNZ8148 (Kuipers, O.P., et al. (1997). "Controlled overproduction of proteins by lactic acid bacteria." Trends Biotech. 15(4): 135-140). The ligation mix was used to transform electrocompetent *Lactococcus lactis* MG1363 and transformants were identified after overnight incubation on M17G agar plates containing 5 µg Cm.ml⁻¹. Twenty colonies were cultured overnight and recombinant plasmids were purified from the pellet using QIAprep Spin Miniprep kit (Qiagen). Insertion site and sequence were confirmed by DNA sequencing. The plasmids were used to transform electrocompetent *S*. *zooepidemicus* cells and recombinant strains isolated after overnight culture on M17G agar plates containing 2.5 µg.ml⁻¹ of Cm. The recombinant strains were routinely maintained on sheep blood agar plates containing 2.5 µg.ml⁻¹ Cm.

**Table 1. Oligonucleotide primers used. Endonuclease restriction sites are underlined.**

| Primer | Sequence (5' - 3') | 5' site |
|---|---|---|
| HasAF | AGTCCATGGAATACAAAGCGCAAGAAAGGAAC | Ncol |
| HasAR | ATCGCATGCCTCCCTTGTCAGAACCTAGG | SphI |
| HasBF | GTCCATGGAAGAAATGAAAATTTCTGTAGCAGG | Ncol |
| HasBR | ATCGCATGCCTAGTCTCTTCCAAAGACATCT | SphI |
| HasCF | GTCCATGGAAGAACTCATGACAAAGGTCAGAAAAG | Ncol |
| HasCR | ATCGCATGCGCTCTGCAATAGCTAAGCCA | SphI |
| GlmUF | GTCCATGGAAAGGAATCAAAACATGAAAAACTACG | Ncol |
| GlmUR | ATCTCTAGAACTATAGCTTACTGGGGCTG | Xbal |
| PgmF | TCGTACCATGGTGAAAGCGGTTAAAAATATAAAGGAG | Ncol |
| PgmR | CCGACTCTGCAGCAGCTCATTTAAGAGACAAAGGCG | Pst I |
| PgiF | GTCCATGGAAGGGAGTAAAATAATGTCACATATTACA | Ncol |
| PgiR | ATCGCATGCTTACAAGCGTGCGTTGA | SphI |
| GuaBF | GTTGATGTGGTTAAGGTTGGTATCGG | - |
| GuaBR | AGCCTTGGAAGTAACGGTCGCTTG | - |

### Growth medium and cultivation conditions

A single colony was selected from the blood agar plate and inoculated overnight in a chemically defined medium (CDM; Table 2). For pNZ-strains, 2.5 µg.ml⁻¹ of Cm and 20 ng.ml⁻¹ of nisin were added to the medium. Growth was monitored at 530 nm with a spectrophotometer.

When an OD₅₃₀ of around 1 was reached, the culture was inoculated to an OD₅₃₀ of 0.05 into a 2L bioreactor (Applikon). The bioreactor was operated at a working volume of 1.4L and the temperature maintained at 37°C. The reactor was agitated at 300 rpm and anaerobic conditions maintained by nitrogen sparging during fermentation. pH was controlled at 6.7 by the addition of 5M NaOH and 5 M HCl.

**Table 2. The chemically defined medium (CDM) used in this study was modified from Van de Rijn, I. et al. (1980). "Growth characteristics of group A streptococci in a new chemically defined medium." Infect. Immun. 27(2): 444-448. All chemicals were purchased from Sigma Aldrich.**

| | Component | Concentration (mg/L) |
|---|---|---|
| 1. | FeSO₄.7H₂O | 10 |
| | Fe(NO₃)₂.9H₂O | 1 |
| | K₂HPO₄ | 200 |
| | KH₂PO₄ | 1000 |
| | MgSO₄.7H₂O | 1000 |
| | MnSO₄ | 10 |
| 2. | Alanine | 200 |
| | Arginine | 200 |
| | Aspartic acid | 200 |
| | Asparagine | 200 |
| | Cystine | 100 |
| | Glutamic acid | 200 |
| | Glutamine | 5600 |
| | Glycine | 200 |
| | Histidine | 200 |
| | Isoleucine | 200 |
| | Leucine | 200 |
| | Lysine | 200 |
| | Methionine | 200 |
| | Phenylalanine | 200 |
| | Proline | 200 |
| | Hydroxy-L-proline | 200 |
| | Serine | 200 |
| | Theonine | 400 |
| | Tryptophan | 200 |
| | Tyrosine | 200 |
| | Valine | 200 |
| 3 | Glucose | 20000 |
| 4. | Uridine | 50 |
| | Adenine | 40 |
| | Guanine | 40 |
| | Uracil | 40 |
| 5. | CaCl₂.6 H₂O | 10 |
| | NaC₂H₃O₂.3H₂O | 4500 |
| | Cysteine | 500 |
| | NaHCO₃ | 2500 |
| | NaH₂PO₄.H₂O | 3195 |
| | Na₂HPO₄ | 7350 |
| 6. | p-Aminobenzoic acid | 0.2 |
| | Biotin | 0.2 |
| | Folic acid | 0.8 |
| | Niacinamide | 1 |
| | B-NAD | 2.5 |
| | Pantothenate Ca salt | 2 |
| | Pyridoxal Hydrochloride | 1 |
| | Pyridoxamine hydrochloride | 1 |
| | Riboflavin | 2 |
| | Thiamine hydrochloride | 1 |
| | Vitamin B12 | 0.1 |
| | Inositol | 2 |

### Measurement of biomass and fermentation products

Samples were collected hourly and the optical density measured with a spectrophotometer at a wavelength of 530 nm and converted to biomass using the equation: Biomass (g/L) = OD530 * 0.26 ± 0.01 (Goh, L.-T. (1998). Fermentation studies of Hyaluronic acid production by *Streptococcus zooepidemicus.* Department of Chemical Engineering. Brisbane Australia). The remaining sample was mixed with an equal volume of SDS to break the HA capsule and filtered through a syringe filter (0.45 µm) for cell removal.

Lactic acid, acetate, formate, glucose and ethanol were measured by HPLC using a BioRad HPX-87 H acid column with 1M H₂SO₄ as eluent and a flow rate of 1 mL per minute. Samples with glucose concentrations below 40 ppm were analysed using a YSI 2700 Select Biochemistry Glucose Analyser (Yellow Springs Inc.).

The concentration of the HA sample was measured using a HA turbidimetric quantification assay (Di Ferrante, N. (1956). "Turbidimetric measurement of acid mucopoly-saccharides and hyaluronidase activity." J. Bio. Chem. 220: 303-306). Briefly, 200 µL of the sample was mixed with 200 µL of 0.1 M potassium acetate (pH=5.6) and 400 µL of 2.5 %w/v cetyl-trimethyl-ammonium-bromide (CTAB) in 0.5 M NaOH. After 20 minutes of incubation, the OD600 was determined and the HA concentration determined from a calibration curve.

### Assay of enzyme activities

*hasA* enzyme assay *hasA* activity was assayed by *in vivo* synthesis of HA from membrane extract obtained using a protocol based on a previously described method (Tlapak-Simmons, V.L., Baggenstoss, B.A., Kumari, K., Heldermon, C., and Weigel, P.H. (1999). Kinetic Characterization of the Recombinant Hyaluronan Synthases from Streptococcus pyogenes and Streptococcus equisimilis. J Biological Chemistry 274, 4246-4253). Initially, 400 µL of membrane lysate, was mixed with 200 µL of 4 mM UDP-Glucuronic acid dissolved in wash buffer (50 mM KH₂PO₄, 5 mM EDTA, 10% Glycerol, protease inhibitors mixture (GE healthcare) , pH 7)) and 400 µL of 4 mM UDP-N-acetyl glucosamine (in wash buffer). Subsequently, 100 µL of HAS buffer (250mM Na₂HPO₄, 250mM KH₂PO₄, 500mM NaCl, 1mM EGTA), 20 µL of 1 M MgCl₂, 20 µL of 20 mM DTT, 10 µL protease inhibitors mixture (GE healthcare) and 50 µL of wash buffer were added to the reactants. The enzymatic reaction was maintained at 37°C in a water bath for 2 hours and subsequently in a 100°C water bath for 2 minutes to terminate the reaction (Tlapak-Simmons, et al. (1999) ibid). After cooling to room temperature , 1 mL of 0.1% SDS was added to free the HA attached to the membrane extract and HA was measured by the Turbidimetric assay described above.

Other enzyme activities were assayed using protocols based on previously described methods: HasB (Dougherty, B. and van de Rijn, I. (1993). "Molecular characterization of hasB from an operon required for hyaluronic acid synthesis in group A streptococci. Demonstration of UDP- glucose dehydrogenase activity." J. Biol. Chem. 268(10): 7118-7124), HasC (Franke, J. and Sussman, M. (1971). "Synthesis of Uridine Diphosphate Glucose Pyrophosphorylase during the Development of Dictyostelium discoideum." J. Biol. Chem. 246(21): 6381-6388), α-phosphoglucomutase (α-Pgm) (Degeest, B. and de Vuyst, L. (2000). "Correlation of activities of the enzymes alpha-phosphoglucomutase, UDP-galactose 4-epimerase, and UDP-glucose pyrophosphorylase with exopolysaccharide biosynthesis by Streptococcus thermophilus LY03." Appl Environ Microbiol 66(8): 3519-27), and Pgi (Bergmeyer, H.U., et al. (1974). Methods of Enzymatic Analysis (Bergmeyer, H.U., ed.). New York, NY, Academic Press, Inc.).

GlmU activity was not determined, but expression was confirmed using real-time PCR. RNA was purified from the cell extracts using the RNeasy mini kit (Qiagen), DNase treated and subjected to RT-PCR with the SuperScript One-Step RT PCR kit (Gibco) using primers: GlmUF3 (5'-ACAATAAGCG CCTCTTTGAG-3') and. GlmUR2 (5'-TCCAACCTTTTCTTGGCTG-3') After 24 cycles, the resultant 360bp DNA fragment of the glmU gene was quantified on an agarose gel.

### Molecular weight determination

HA samples were purified from the broth by mixing 15mL of culture with 15mL of 0.1% w/v SDS incubated at room temperature for 10 minutes (Chong, B.F. (2002). Improving the cellular economy of *Streptococcus zooepidemicus* through metabolic engineering. Department of Chemical Engineering. Brisbane, The University of Queensland). Samples were then filtered through a 0.45µm filter and the filtrates were thawed and mixed with 3 volumes of ethanol and left overnight at 4°C. The precipitates were then centrifuged (9630 × g; 4°C; 20 min) and supernatant removed. The pellet was washed in 15 mL ethanol; saline solution (75% w/v ethanol, 25 % w/v 0.15M NaCl) and again centrifuged (17600 × g; 4°C; 20 min). After removal of the supernatant, the pellet was allowed to dry overnight. Finally, the HA pellet was then resuspended in 0.15 M NaCl with gentle rocking and undissolved matter was removed by centrifugation (17600 xg; 4°C; 20 min) and samples were filtered through 0.45µm filter.
Intrinsic viscosity was measured with a Lauda Processor viscosity measuring system using an Ubbelohde Dilution Capillary (0.63 mm diameter, 5700 mm³ volume). All measurements were performed at 37°C and 0.15M sodium chloride was used as diluting solvent. The intrinsic viscosity was used to determine the average molecular weight using the Mark-Houwink-Sakurada equation: [η]=0.02×*M̅_{w}*^{0.6479}, with parameters fitted using standards of known molecular weight processed as outlined above.

### Results

Six genetically modified S. *equi* strains were generated as outlined in Materials and Methods. Overexpression of each of the 6 genes was confirmed using enzyme assays (*hasA, hasB, hasC, pgm* or *pgi*) or RT-PCR (*glmU*). Each strain was fermented in a bioreactor and the molecular weight of HA produced determined using viscometry. By overexpressing the genes *hasB, pgm, pgi* and *glmU,* we were able to increase the MW of the product by 22%, 47%, 82% and 47% respectively compared to the WT. Surprisingly, overexpression of hasA (HA synthase) had no significant effect (Table 3).

**Table 3 - % increase in molecular weight of HA produced by genetically modified S. equi strains (relative to wild type value of 2.015 MDa)**

| **gene** | **% increase in HA MW relative to wildtype** |
|---|---|
| hasA | 2% |
| hasB | 22% |
| hasC | 5% |
| glmU | 47% |
| pgm | 56% |
| pgi | 82% |

### Conclusion

We describe the design and construction of a number of streptococcal strains that overexpress specific enzymes in the HA biosynthetic pathway, and which are capable of synthesizing significantly higher MW HA comparing to wild type strains.

The various features and embodiments of the present invention, referred to in individual sections above apply, as appropriate, to other sections, *mutatis mutandis.* Consequently features specified in one section may be combined with features specified in other sections as appropriate.

All publications mentioned in the above-specification are herein incorporated by reference. Various modifications and variations of the described methods and products of the invention will be apparent to those of skill in the art without departing from the spirit and scope of the invention. Although the invention has been described in connection with specific preferred embodiments, it should be understood that that the invention as claimed should not be unduly limited to such specific embodiments. Indeed, in various modifications of the described modes for carrying out the invention which are apparent to those skilled in the relevant fields are intended to be within the scope of the following claims.

## Claims

1. A method for producing hyaluronic acid which method comprises growing *Streptococcus* cells in a culture medium, which cells express the enzymes required for hyaluronic acid synthesis, wherein the activity in the cells of one or more enzymes selected from UDP-glucose dehydrogenase, phosphoglucoisomerase, glucosamine-6- phosphate acetyl transferase/N-acetyl glucosamine-1-phosphate pyrophosphorylase and phosphoglucomutase has been increased; and optionally recovering the hyaluronic acid produced by the cells.

2. A method for producing hyaluronic acid which method comprises growing *Streptococcus* cells in a culture medium, which cells express the enzymes required for hyaluronic acid synthesis, wherein the cells have been engineered or treated to increase the activity of one or more enzymes selected from UDP-glucose dehydrogenase, phosphoglucoisomerase, glucosamine-6- phosphate acetyl transferase/N-acetyl glucosamine-1-phosphate pyrophosphorylase and phosphoglucomutase; and optionally recovering the hyaluronic acid produced by the cells.

3. A method according to claim 1 or claim 2 wherein the cells have been transformed with one or more nucleic acid constructs which encode said one or more enzymes.

4. A method according to any one of claims 1 to 3 which further comprises recovering the hyaluronic acid produced by the cells.

5. A method for producing hyaluronic acid which comprises recovering hyaluronic acid from *Streptococcus* cells that express the enzymes required for hyaluronic acid synthesis, wherein the activity in the cells of one or more enzymes selected from UDP-glucose dehydrogenase, phosphoglucoisomerase, glucosamine-6- phosphate acetyl transferase/N-acetylglucosamine-1-phosphate pyrophosphorylase and phosphoglucomutase has been increased.

6. A method for producing hyaluronic acid which comprises recovering hyaluronic acid from *Streptococcus* cells that express the enzymes required for hyaluronic acid synthesis, wherein the cells have been engineered or treated to increase the activity in the cells of one or more enzymes selected from UDP-glucose dehydrogenase phosphoglucoisomerase, glucosamine-6- phosphate acetyl transferase/N-acetyl glucosamine-1-phosphate pyrophosphorylase and phosphoglucomutase.

7. Purified bacterial hyaluronic acid produced by the method of any one of claims 1 to 6.

8. A composition comprising purified bacterial hyaluronic acid according to claim 7.

9. A *Streptococcus* cell which comprises the enzymes for synthesis of hyaluronic acid, which cell has been genetically modified to overexpress one or more enzymes selected from UDP-glucose dehydrogenase, phosphoglucoisomerase, glucosamine-6- phosphate acetyl transferase/N-acetyl glucosamine-1-phosphate pyrophosphorylase and phosphoglucomutase.

10. Use of a cell according to claim 9 in a method of producing hyaluronic acid.
